Europäisches Patentamt

European Patent Office

Office européen des brevets

(19)

(11) Publication number: **0 374 665**
**A2**

# EUROPEAN PATENT APPLICATION

(12)

(21) Application number: 89122786.0

(51) Int. Cl.5: **C12Q 1/68**

(22) Date of filing: 09.12.89

(30) Priority: **23.12.88 US 289638**

(43) Date of publication of application:
**27.06.90 Bulletin 90/26**

(84) Designated Contracting States:
**AT BE CH DE ES FR GB GR IT LI LU NL SE**

(71) Applicant: **MOLECULAR DIAGNOSTICS, INC.**
**400 Morgan Lane**
**West Haven, CT 06516(US)**

(72) Inventor: **Dattagupta, Nanibhushan**
**470 Prospect Street**
**New Haven, CT 06511(US)**

(74) Representative: **Dänner, Klaus, Dr. et al**
**c/o Bayer AG Konzernverwaltung RP Patente**
**Konzern**
**D-5090 Leverkusen 1 Bayerwerk(DE)**

(54) Assay of sequences using amplified genes.

(57) A method for amplifying specific target nu leic acid sequences in a sample, the method comprising
· (a) contacting under hybridization conditional (i) a first primer nucleic acid and a second primer nucleic acids, one of said first or second primer nucleic acids being immobilized or immobilizable, the other is either immobilized or immobilizable or labeled, with (ii) a test sample containing target nucleic acid sequences,

(b) contacting the resultant product of step (a) with an extender enzyme and at least one nucleotide residue for extension under conditions to elongate the resultant hybridized primer nucleic acids, and

(c) denaturing the product of step (b), to produce immobilized target nucleic acid sequences, and

(d) repeating at least once a cycle of steps (a), (b) and (c) with the product of step (c) of the previous cycle being used in place of (a)(ii). The above described method can be used to detect specific target nucleic acid sequences by using a test sample suspected of containing target nucleic acid sequences and determining if an amplified sequence is present.

EP 0 374 665 A2

EP 0 374 665 A2

## ASSAY OF SEQUENCES USING AMPLIFIED GENES

### BACKGROUND OF THE INVENTION

Field of the Invention

The present invention relates to the amplification of a gene for the detection of specific sequences. More particularly, the present invention concerns a method of detecting a specific nucleic acid sequence using immobilized or immobilizable oligonucleotides.

Background Information

Large scale production of a nucleic acid sequence is usually done by cloning the particular sequence in a specific vector. The sequence to be cloned is isolated, identified, coupled covalently to a single or double-stranded vector and then cloned. The vectors with the extra DNA are separated from the host cell and, depending on the requirements, the cloned piece of DNA has to be restricted and separated from the rest of the DNA. If one requires single-stranded DNA, either it is cloned in a single-stranded vector or strand separation is necessary. All these techniques involve skilled manipulation of biochemical and biological systems.

Analytical Biochemistry, 140, 95-103, (1984) describes a method of producing DNA hybridization probes by non-specifically immobilizing a single strand DNA template to cellulose. Although the method is useful, the length distribution of the newly synthesized product DNA is not as uniform as might be desired. It now appears this may be due to multiple attachments of the template DNA to the cellulose.

A homogenous system involving two probes has been described in European patent application No. 192,168. This method uses two non-overlapping probes, one of which is labeled for detection and the other for the separation of the hybrid. The assay takes place in a homogeneous solution and the hybrid is subsequently separated by an immobilization reaction with a solid support and a separation probe.

Amplification of purified polynucleotide sequences by using an immobilized polynucleotide and oligonucleotide as a primer has been described in U.S. Patent 4,734,363 and in Ashley et al, Anal. Biochem., supra. In U.S. Patent 4,734,363 an end coupling and in Ashley et al nonspecific random coupling reactions were used for the immobilization of the polynucleotides.

Saiki et al, Science, 230, 1350, (1985) described a method of amplification of a beta-globin nucleic acid sequence in a human genomic sample for the detection of point mutation by hybridization with an oligonucleotide. This product sequence is formed in solution. For hybridization, the DNA has to be either immobilized after amplification or a restriction digestion and separation must be carried out for analysis of the sequence.

The method of amplification described by Saiki et al, is also described in U.S. Patent 4,683,195 and U.S. Patent 4,683,202 (hereinafter Mullis). A significant limitation of this method is that the resulting amplified sequence cannot be readily separated from the mixture or manipulated, and thus requires significant further efforts to analyze or detect the amplified sequence at the termination of thermocycling.

### SUMMARY OF THE INVENTION

The present invention relates to the amplification of a gene for the detection of specific sequences. The amplified sequences are produced by using immobilized or immobilizable or nonisotopically labeled primer sequences so that the product can be immobilized for detection. The sequences which serve as primers can also be labeled during the extension reaction.

More particularly, the present invention concerns a method for amplifying specific target nucleic acid sequences in a sample, for example, in a sample comprising double stranded DNA, the method comprising

(a) a priming step involving contacting under hybridization conditions (i) a first primer nucleic acid and a second primer nucleic acid, one of the first or second primer nucleic acids being immobilized or immobilizable, the other is either immobilized or immobilizable or labeled, with (ii) a test sample containing

2

target nucleic acid sequences,

(b) contacting the resultant produce of step (a) with an extender enzyme and at least one nucleic acid residue under conditions to elongate the resultant hybridized primer nucleic acids,

(c) denaturing the product of step (b) to produce immobilized target nucleic acid sequences, and

(d) repeating at least once a cycle of steps (a), (b) and (c) with the product of step (c) of the previous cycle being used in place of (a)(ii).

The present invention also concerns a method for detecting specific target nucleic acid sequences in a sample, e.g., the sample comprising double stranded DNA, the method comprising

(a) contacting under hybridization conditions (i) a first primer nucleic acid and a second primer nucleic acid, one of the first or second primer nucleic acids being immobilized or immobilizable, the other is either immobilized or immobilizable or labeled, with (ii) a test sample suspected of containing target nucleic acid sequences,

(b) contacting the resultant product of step (a) with an extender enzyme and at least one nucleic acid residue under conditions to elongate the resultant hybridized primer nucleic acids,

(c) denaturing the product of step (b) to produce immobilized target nucleic acid sequences,

(d) repeating at least once a cycle of steps (a), (b) and (c) with the product of step (c) of the previous cycle in place of (a)(ii), and

(e) determining if an amplified sequence is present.

The present invention further concerns a kit for amplifying specific target nucleic acid sequences, the kit comprising one or more containers containing (a) an immobilized or immobilizable primer nucleic acid, (b) an immobilized, immobilizable or labeled nucleic acid, (c) an extender enzyme and (d) at least one nucleotide residue for extension.

The present invention also is directed to a kit for detecting specific target nucleic acid sequences, the kit comprising one or more containers containing (a) an immobilized or immobilizable nucleic acid, (b) an immobilized, immobilizable or labeled nucleic acid, (c) an extender enzyme, (d) at least one nucleotide residue for extension and (e) a labeled hybridizable oligonucleotide specific for the detection of the amplified sequence.

The present invention provides a significant improvement over the prior art in that an immobilized or immobilizable oligonucleotide can be used for amplification of a specific sequence in a crude mixture containing many other sequences. Moreover, the desired analyte produced at the end of the process is in immobilized form and ready for heterogeneous phase assays. The present invention produces immobilized amplified sequences and can be processed as an automated system for complete analysis.

One embodiment of the present invention is an improvement over the Mullis patent, U.S. Patent 4,683,195 and U.S. Patent 4,683,202, incorporated herein by reference. In this improvement, at least one of the primers is immobilized or immobilizable.

## BRIEF DESCRIPTION OF THE DRAWING

Fig. 1 is a schematic diagram showing the steps to carry out an amplification according to an embodiment of the present invention.

Fig. 2 is a photograph of the results of amplification of nucelic acids according to the invention as analyzed by gel electrophoresis.

Fig. 3 is a photograph of the results of restriction endonuclease digestion of the products of amplification according to the present invention. Fig. 3 shows that the inventive method produces results as specific as if conducted in solution.

Fig. 4 is a photograph of two test tubes containing specific amplified (CL = chlamydia) product and control (mouse).

## DETAILED DESCRIPTION OF THE INVENTION

Several preferred embodiments of the present invention are summarized as follows:

3

TABLE 1

| Nature of Primer Nucleic Acid 1 | Nature of Primer Nucleic Acid 2 | Detection of the Amplified Product |
|---|---|---|
| (1) immobilized | immobilized | agglutination, hybridization, colorimetric, or fluorometric |
| (2) immobilized | immobilizable | agglutination, hybridization, colorimetric, or fluorometric |
| (3) immobilized | labelled | label or hybridization |
| (4) immobilizable | labelled | label or hybridization |
| (5) immobilizable | immobilized | agglutination, hybridization, colorimetric, or fluorometric |
| (6) labelled | immobilized | label or hybridization |
| (7) labelled | immobilizable | label or hybridization |
| (8) immobilized/labelled | | agglutination, hybridization, colorimetric, or fluorometric |
| (9) immobilizable/labelled | | agglutination, hybridization, colorimetric, or fluorometric |
| (10) | immobilized/labelled | agglutination, hybridization, colorimetric, or fluorometric |
| (11) | immobilizable/labelled | agglutination, hybridization, colorimetric, or fluorometric |

In Table 1, it is to be understood that other methods of detection of the amplified product can be employed, e.g., restriction endonuclease digestion of the product and gel electrophoresis.

A detailed description of particular embodiments (3) and (6) in the above Table 1 concerning amplification of a test nucleic acid sequence for an assay involves contacting a nucleic acid attached to a solid support complementary to one strand of the test nucleic acid sequence and another nucleic acid attached to a detectable label, the nucleic acid being conplementary to another strand of the test sample nucleic acid under hybridization conditions, extending the hybridized nucleic acid with an enzyme such that sequences complementary to the test sample are produced, denaturing the product and reannealing to produce a more extendable structure and repeating the process to produce a solid support immobilized test sequence in an amount greater than the starting concentration. The product is then assayed, for example, as follows:

(a) if a labeled oligonucleotide is used as a primer and the second primer is in an immobilized form, detection of the label on the support will determine the presence of the test amplified nucleic acids;

(b) by hybridization with a specific probe;

(c) if during an extension reaction a labeled nucleic acid residue is incorporated, the extent of incorporation of a such a residue determines the presence of the specific sequence; or

(d) a post extension agglutination reaction.

A detailed description of other embodiments (4) and (7) in the above Table 1 concerning amplification of a nucleic acid sequence for an assay involves contacting a nucleic acid immobilizable to a solid support complementary to one strand of the test nucleic acid sequence and another nucleic acid attached to a detectable label, the nucleic acid being complementary to another strand of the test sample nucleic acid under hybridization conditions, extending the nucleic acid with an enzyme such that sequences complementary to the test sample are produced, denaturing the product, reannealing to produce a more extendable structure, repeating the process to produce a test sequence in an amount greater than the starting concentration and contacting the amplified product with a solid support to effect immobilization for detection. The immobilized product is then assayed, for example, as follows:

(a) detecting label on the solid support; and

(b) by hybridization with a specific probe.

In a similar manner embodiments (1), (2) and (5) of Table 1 can be carried out.

As described above, Saiki et al and Mullis describe a polymerase chain reaction (PCR) method of amplification of a test sample. Saiki et al and Mullis used two different oligonucleotides as primers. The test sample nucleic acid served as a template for the primer extension reaction. The oligonucleotides were complementary to the ends of the two strands of a double stranded sample DNA. In Saiki et al and Mullis, one oligonucleotide is specific for one strand and the other oligonucleotide is specific for the complementary strand. Both oligonucleotides flank the region of mutation sequence to be analyzed. When the oligonucleotides are mixed with the test sample nucleic acids under annealing conditions, the oligonucleotides will hybridize in a fashion such that the 3' hydroxyl of each oligonucleotide will be available for extension. After the oligonucleotide is extended with template dependent primer extension enzyme, e.g., DNA, polymerase, Reverse transcriptase etc., the processes of oligonucleotide hybridization and extension are repeated. A disadvantage of such an amplification is that the final product analysis requires difficult sample handling steps, e.g., immobilizing.

It has been found that the PCR method is significantly improved by the use of immobilized or immobilizable nucleic acid primers. The final amplified products of the present invention are already immobilized or specifically immobilizable without significant loss in efficiency of amplification.

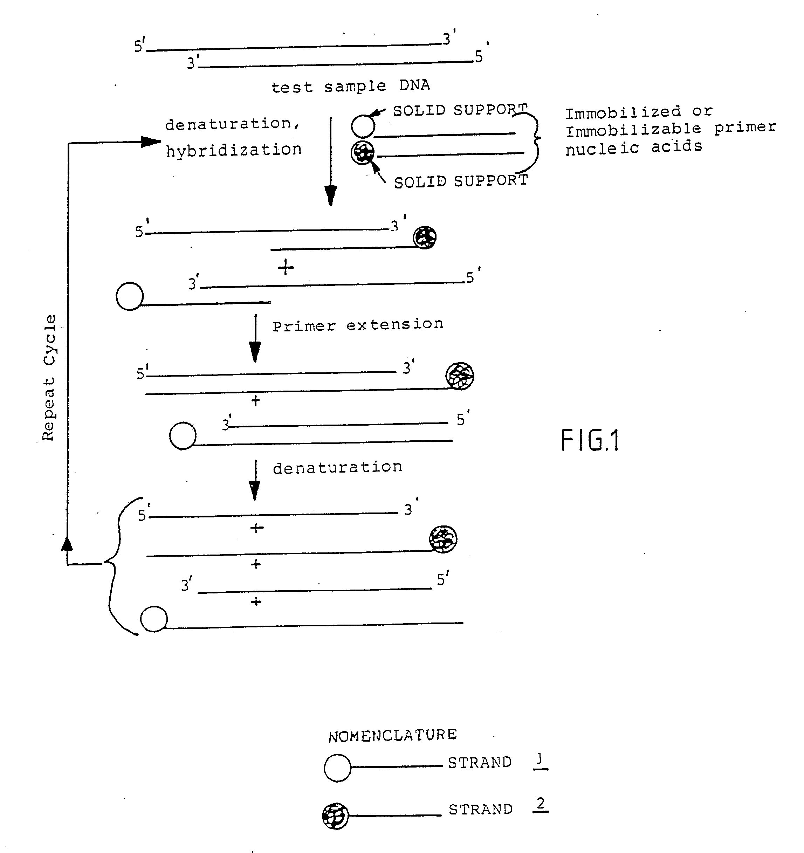

In Fig. 1, the test sample nucleic acid, which serves as a template for the initial cycle of amplification, is contacted with immobilized or immobilizable primer nucleic acids under conditions of hybridization. One primer sequence is specific for one strand and the other is specific for the complementary strand. The primers are not complementary to each other. After the primer template hybrids are formed they are extended by an enzyme. The amplified sequence is then recycled for further amplification.

In a preferred embodiment, the primer nucleic acids are oligonucleotides and they are complementary to the 3' end sequences of the sample DNA. The nucleic acid primers can be of any length between 3 and 10 kilobases and greater, preferably between 5 and 100 bases. Any complenentary (to the sample) nucleic acids with 3'-hydroxy can serve as primer for template mediated extension reaction.

The objective of using two primers is to amplify both strands. The primers do not have to be complementary (in their entirety), to the sample sequence. They should be complementary enough to initiate sequence specific, template mediated extension reaction. In order to maintain the fidelity of the template mediated primer extension reaction the initiation should occur from a perfectly matched base paired region. The minimum sequence may include 3 base pairs. Usually a 5 to 7 base pair double stranded region is strong enough to undergo primer extension reaction at 25° C and below. If it is less than 5 bp, a low temperature extension reaction has been carried out.

The present invention is an improvement over the amplification methods described in the prior art. EP 0192,168 discloses methods for immobilizing and detecting polynucleotide sequences and is incorporated herein by reference. The present invention describes a method of amplification of test sample nucleic acids where the amplified sequence is produced in an immobilized form. The major advantage associated with such process is that the product is formed in a specifically immobilizable or immobilized state. This makes the separation from the unamplified sequence (sometimes $10^3$-$10^6$ times the test sequence) easier. Since it is in a specifically immobilizable or immobilized form the analysis is easier.

Immobilization of a nucleic acid, especially an oligonucleotide is described in Affinity Chromatography, Herbert Schott, Marcel Dekker, Inc., pages 15 to 21 (1984). Most of the immobilization reactions described therein can be carried out via spacer or linker residues. For example, instead of using "SEPHADEX" or cellulose particles directly, it is possible to use polyamine or protein linked "SEPHADEX" or cellulose and use the protein or polyamine residues as the coupling sites for an oligonucleotide.

For example, cyanogen bromide activated "SEPHADEX" can first be reacted with a large excess of a polyamine, e.g., spermine, and then coupled to an oligonucleotide by an oxidative reaction. As has been described in EP 164,586, aminolabeled oligonucleotides can be prepared with variable linker lengths. Other methods for preparing aminolabeled oligonucleotides have also been described in the art.

As an immobilization matrix (solid support) for the present invention reactive or activated cellulose, "SEPHADEX", "SEPHAROSE", "SEPHACRYL", polystyrene latex, polyacrylates, polyvinylalcohols, or other synthetic or naturally occurring matrices which can be activation to undergo chemical reaction.

The substrate for the extension reaction is one or more nucleic acid residues, e.g., nucleoside triphosphate (NTP).

Since the addition of nucleoside residues to the primer or extension reaction necessary for the fidelity of the sequence should be specific, enzymes such as DNA polymerase, a Klenow fragment of polymerase or reverse transcriptase, tag polymerase are preferable. These enzymes catalyze a specific primer extension reaction. It was unexpected and not predictable that such enzymes would act on oligonucleotide primers when they are immobilized without significant loss of efficiency.

The present invention produces immobilized sequences for analysis. In one embodiment, the presence of such a sequence can be detected by using labeled nucleotide residues as substrates for extension. As, for example, if a $^{32}$P-labeled or fluorescein labeled nucleoside triphosphate is used as a substrate for extension with an enzyme DNA polymerase the extended product will be labeled. By detecting such labels (radioactive for $^{32}$P and fluorescence for fluorescein) it is possible to ascertain the presence of the test sequence nucleic acid.

The amplified signal can also be detected by hybridization with a specific probe.

The immobilized or immobilizable oligonucleotide probes will comprise at least one single stranded base sequence complementary to the 3' end or the 5' end of the sequence to be amplified. The probe should not be complementary to the entire sequence before the enzymatic extension reaction is conducted. The homology of the ends of the probe can be as short as three nucleotides and can be as long as 200 nucleotides, however, between 10 and 30 nucleotide residues is preferred.

The oligonucleotides can be immobilized by virtue of the oligonucleotides being linked at one end thereof to a solid substrate and wherein the elongation occurs in a direction opposite to the solid substrate.

The sample can comprise double stranded DNA and each oligonucleotide can be complementary to a strand of the double stranded DNA.

Detection of the presence of the amplified sequence can be conducted in many ways including any of the following:

(1) labeling the nucleic acid residue, for example, with a label, such as, for example, a radioactive moiety, a dyestuff, a fluorescent moiety or an enzyme, and then conducting an analysis to determine the presence of such label;

(2) subjecting the product of step (d) of the aforementioned method for detecting specific target nucleic acid sequences to hybridization conditions with a labeled probe (such label can be as described hereinabove) hybridizable with the sequence being tested for and determining if in fact hybridization has occurred;

(3) determination of a change or difference in a physical property, e.g., to detect a difference in molecular weight or density by conducting electrophoresis, centrifugation, gel permeation chromatography or using microscopy (visual determination to detect a difference in size).

## Primers:

Primers are known in the art as the nucleic acid molecules which can be enzymatically extended when they form a duplex with a complementary nucleic acid with an extra single stranded (which can be present or can be created in the reaction) sequence in a proper orientation. Since most of the nucleic acid extending enzymes add residues to the 3' side of the exisiting nucleoside residue, the copying sequence should be single stranded in a complementary form towards the 3' side of the primer. Primers are usually oligonucleotides but include any hybridizable DNA, RNA or olignucleotide. A higher molecular weight single stranded nucleic acid can also serve as a primer. Nucleic acids as short as a trinucleotide and as long as 10kb can serve as a primer. The preferable size is determined by the specific nature of the sequence to be copied or amplified. The most preferable sizes for the primers are between an hexanucleotide and a thirtymer. The examples in this application are with oligonucleotide primers of the lengths between 20mers to 30mers, but other lengths can be utilized. Immobilizable nucleic acids have been described in detail in EP 192,168. The extended primers can be used in a similar fashion.

## Immobilization Methods:

Chemical immobilization of an oligonucleotide is well known in the art. As for example, Affinity Chromatography by H. Schoot, Marcel Dekker, 1984, decribes different methods of immobilization of oligonucleotides and nucleic acids. Other methods which are not in that book are also known in the art. A copolymer of chloromethyl styrene and styrene can be used for the immobilization of a primary amine containing olignoucleotide. Different types of latex particles can be used for immobilization following similar chemistry as used in protein immobilization. In principle, solid supports such as cellulose and its derivatives, "SEPHADEX", "SEPHAROSE" and similar materials, latex particles of papers, magnetic or nonmagnetic particles, silica based particles, glass based materials, polyurethanes, "TEFLON" based particles or sheets, "IMMOBILON" and others, just to name a few, can be used as solid supports for immobilization.

If magnetic particles are used, the separation of unbound fluorescent primers from fluorescent-bound primers can be effected by the use of a magnet field. After the amplication procedure, the reaction chambers are held close to a magnetic field. The immobilized particles are fixed and the supernatant can be decanted or aspirated. The immobilized particles can be repeatedly washed to remove any excess unbound fluorescent-labeled primers from the reaction mixture. After washing the magnetic particles can be resuspended and the fluorescent signal on the particles can be determined qualitatively or quantitatively. Other labeling methods can be utilized in combination with magnetic particles.

Labelling and Detection Without Hybridization:

When one of the primers is immobilized or immobilizable, the detection can be carried out on the solid support either by hybridization with a labelled probe or the product can be directly detected by using the other primer derivatized with a detectable label. The labelling of an oligonucleotide with a detectable label, as for example, with a fluorophore is known in the art. All the detection techniques can be carried out by a skilled artisan.

Biotin can be utilized as a label for an immobilized or immobilizable primer using well known biotin-avidin technology. In an immobilized/labelled system the biotin would be present on one primer and an immobilized substrate on the second primer. In an immobilizable/labelled system, the biotin would be present on one primer and a label such as fluorescein would be on the second primer, following amplification by thermocycling, the biotin containing product could be immobilized.

Labelled Primers:

There are a variety of methods that can be used in the present invention for determining the presence of the labelled extended primer in the separated immobilized fraction or in the remaining reaction solution in order to conclude the assay. One of ordinary skill in the art can choose from any conventional means for detecting the occurrence of a label in the amplified sample.

The label will be a native characteristic of the primer oligonucleotide comprised in the probe or a substance which has a detectable physical, chemical, or electrical property. When a detectable labeling substance is introduced, it can be linked directly such as by covalent bonds to the probe or can be linked indirectly such as by incorporation of the ultimatley detectable substance in a microcapsule or liposome which in turn is linked to the detectable probe.

Labeling materials have been well-developed in the field of immunoassays and in general most any label useful in such methods can be applied to the present invention. Particularly useful are enzymatically active groups, such as enyzmes (see Clin. Chem., (1976) 22:1232, U.S. Reissue Pat. No. 31,006, and UK Pat. 2,019,408), enzyme substrates (see U.S. Pat. No. 4,492,751), coenzymes (see U.S. Pat. Nos. 4,230,797 and 4,238,565), and enzyme inhibitors (see U.S. Pat. No. 4,134,792); fluorescers (see Clin. Chem., (1979) 25:353); chromophores; luminescers such as chemiluminescers and bioluminescers (see U.S. Pat. 4,380,580); specifically bindable ligands such as biotin (see Euroepan Pat. Spec. 63,879) or a hapten (see PCT Publ. 83-2286); and radioisotopes such as $^3$H $^{35}$S, $^{32}$P, $^{125}$I, and $^{14}$C. Such labels are detected on the basis of their own physical properties (e.g., fluorescers, chromophores and radioisotopes) or their reactive or binding properties (e.g., ligands, enzymes, substrates, coenzymes and inhibitors). For example, a cofactor-labeled species can be detected by adding the enzyme (or enzyme where a cycling system is used) for which the label is a cofactor and a substrate or substrates for the enzyme. A hapten or ligand (e.g., biotin) labeled species can be detected by adding an antibody to the hapten or a protein (e.g., avidin) which binds the ligand, tagged with a detectable molecule. Such detectable molecule can be some molecule with a measurable physical property (e.g., fluorescence or absorbance) or a participant in an enzyme reaction (e.g., see above list). For example, one can use an enzyme which acts upon a substrate to generate a product with a measurable physical property. Examples of the latter include, but are not limited to, beta-galactosidase, alkaline phosphatase and peroxidase.

Methods for preparing a label primer used in a preferred embodiment of the present invention are readily available from the prior art. When labeling one will employ synthetic approaches which are effective for modifying nucleic acids without substantially interfering with the ability of the labeled primers to participate in hybridization and extension, and will select labels which are sufficiently stable under the conditions to be used for extension and subsequent detection. For methods on agglutination detection see Grieco and Meriney, Immunodiagnosis for Clinicians (1983), Chapter 2.

The invention will now be described with reference to the following non-limiting examples.

Example 1:

7

The sequence of the human beta-globin gene is used as a model system for this invention. Any known sequence can be amplified by proper selection of the immobilized primers.

A sequence WP or shorter

WP    5′ ACACAACTGTGTTCACTAGC 3′ and a sequence CP or shorter

CP    3′ CCACTTGCACCTACTTCAAC 5′

These oligonucleotides and their relationship to the target globin sequences have been published by Saiki et al, Science, 230, 1350, (1985).

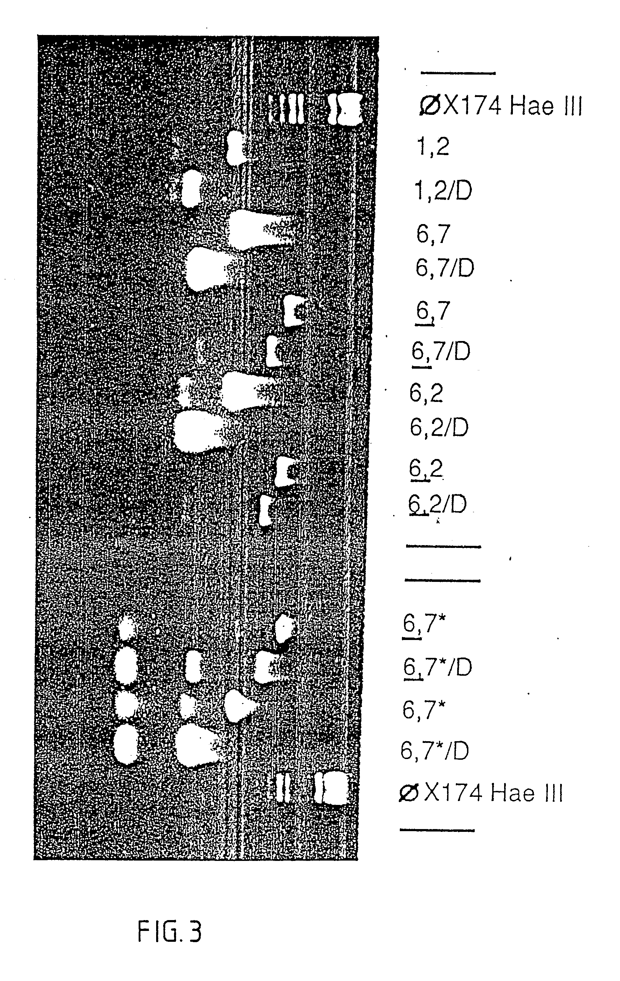

The oligonucleotide primers are modified by placing a carbon chain linker with an amine terminus on the 5′ end of each olignoucleotide using the reagents and protocol available from Applied Biosystems, supra. The modified primer can then be covalently bound to a 3-dimensional surface. For the immobilized solid support the local concentration of the primer is greater than the average concentration in solution. Prior to covalently binding the modified oligonucleotide primers to the solid substrate, the modified primers were subjected to a thermocycling reaction to determine whether the modification of the primers would adversely affect the amplification. As shown in Figure 3, formation of the 110 base pair sequence defined by the primers was not adversely affected by the presence of a $NH_2$ at the 5′ end of the primers. Alternative linkers such as an alkyl, e.g., $C_2$ or $C_{12}$, may be utilized. It is also recognized that the same or different linkers may be utilized to immobilize and/or label the primers. The selection of linkers is based on the absence of interference of amplification of the nucleotide sequence. For example, when the transcription is away from the solid support, shorter linkers may be used. Longer linkers for some solid supports may be necessary when transcription is in the direction of the solid support.

The modified oligonucleotides were covalently bound to chloromethylstyrene(CMS)-beads in the presence of borate buffer. The beads are washed and pelleted to remove any unbound oligonucleotide.

The beads approximately 200 μl in aqueous suspension containing immobilized WP and CP are added to a solution (1 ml) containing 10 mM tris pH 7.5, 50 mM NaCl, 10 mM $MgCl_2$, 1.5 mM deoxynucleoside triphosphates (all four). They are heated to 37°C for 10 minutes and 20 units of Klenow fragment of E. coli DNA polymerase (PL-Biochemical) is added. The reaction is allowed to proceed for 5 minutes. Then the solution is centrifuged in a microfuge and using one of the deoxynucleoside triphosphate as the radioactive substrate the background reaction is estimated by counting the radioactivity of the beads in a scintilation counter. The reaction is repeated after adding (0.1 μg) 10 μl aqueous heat denatured (100°C) ice cooled sample DNA (human). The cycle is repeated by heating the whole mixture in a boiling water bath and rapidly cooling to 37°C. For every cycle an aliquot of fresh enzyme (2 units) is added.

Repeated heating and cooling may result in clumping or aggregation of the beads, leaving them in a less than optimal state for extension and annealing. Methods known in the art to minimize this problem include the use of such materials as "TWEEN 80", glycine, and bovine serum albumin (BSA). One or a combination of such materials added after the modified covalent binding of the oligonucleotide can resolve this problem.

The final product is collected as immobilized amplified sequence by centrifugation in a microfuge. The immobilized product nucleic acid is then assayed in three different ways, namely, as follows:.

    (1) hybridization with a labeled 19 mer;

    (2) digestion with two enzymes (Science, 230, 1350 (1985));

    (3) direct detection with or without any restriction digestion.

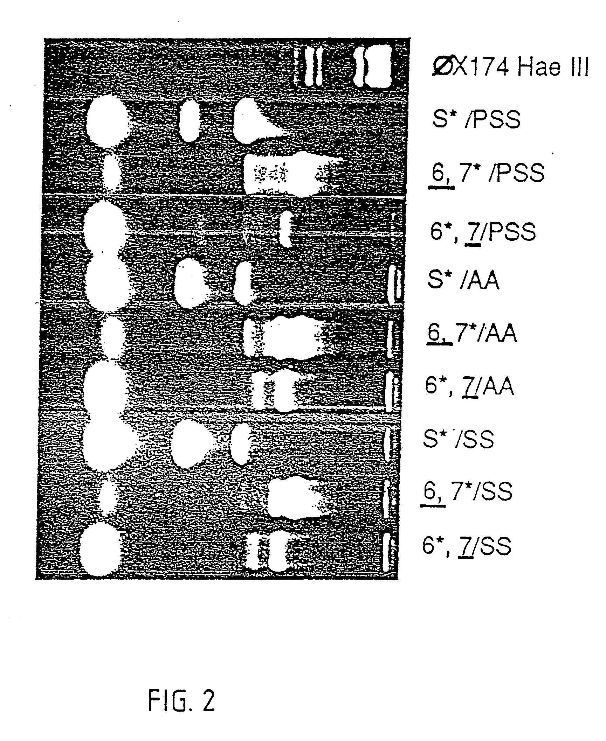

The results of the amplification as analyzed by electrophoresis on an agarose gel are depicted in Fig. 2.

Example 2: Hybridization With a Labeled 19 mer Hybridizable With Labeled Oligonucleotide Probes

The amplified product immobilized onto beads is denatured by heating on a boiling water bath and then chilled in ice. As is described below, the beads are then hybridized with $^{32}$P-labeled oligonucleotides.

For molecular hybridization, the following mixture is prepared:

450 μl 20X NET buffer

750 μl 20% dextran sulfate

150 μl deionized water

75 μl 100X Denhardt's solution

75 μl 10% NP-40 detergent (Sigma)

To this mixture is added a one tenth portion of the oligonucleotide probe prepared as described in N. Dattagupta, D. Rabin, G. Michaud and P. M. M. Rae, "A Simple Method for Generation of High Specific Activity Oligonucleotide Probes", BioTechniques, Vol. 5, No. 1, 38-43, (1987). The final 19-mer probe concentration is approximately 1 nanomolar. It has been found that addition of more probe than this results in an increased background. To prepare a labeled probe for a single or a few reactions, the labeling protocol detailed above can be scaled down, using proportionally less primer, template, dGTP, and dATP. A 0.1X reaction can be done in 10μl, a 0.2X reaction can be done in 20 μl, etc. When volume permits, the [alpha$^{32}$p]-alpha-ATP need not be evaporated to dryness.

Beads containing amplified approximately 0.5 ml of the sequence are placed in plastic bags (e.g., "SEAL-A-MEAL", "SEAL AND SAVE", etc) with one end left open. The radioactive hybridization mix is added with a Pasteur pipet or a syringe with a 21 gauge needle and the remainder end of the bag is sealed. Hybridization is conducted for one hour at 50°C.

After hybridization, the beads are separated by centrifugation in test tubes and washed with 6X SSC (saline sodium citrate) for 15 minutes with gentle shaking.

For stringency washing, the beads are transferred to a prewarmed 1.5 ml culture tube containing 1.4 ml 6X SSC. The tubes are replaced in a 57°C circulating water bath for 10 minutes. The liquid is centrifuged quickly, another 1.5 ml prewarmed 6X SSC is added, the tubes are then replaced in the water bath for another 10 minutes, the liquid is centrifuged again and the supernatant liquid is then discarded.

The radioactivity in the tube is counted in a scintillation counter. The radioactivity associated with the beads is the measure of hybridization and hence the indication of the presence of test gene in the original sample.

## Example 3: Digestion With Two Enzymes

As has been described by Saiki et al, Science, 230, 1350 (1985), the amplified product of Example 1 can be assayed by hybridization and then restriction digestion and separation by gel electrophoresis or thin layer chromatography.

## Example 4: Direct Detection

The amplified product can be directly assayed by using a label carrying nucleoside triphosphate as the substrate for the amplification/extension reaction. As for example if a $^{32}$P-labeled or a fluorescein labeled or a biotin labeled nucleoside triphosphate is used as the substrate, the incorporation of the labels into the extended nucleic acids will be the direct indication of the specific processes and hence the presence of a specific test sequence. After the amplification, the amplified nucleic acids will be in beads and the presence of any label on the beads will indicate the presence of the test sequence. The assays for fluorescein, biotin or $^{32}$P are well known in the art. Other common labels can also be used for this purpose.

The difference in the physical properties of the beads can also be used for detection. Electrophoretic mobility, density in a centrifugation field and chromatographic properties can also be used for detection purposes.

## Example 5: Immobilization of an Oligonucelotide to a Polystyrene Latex

Polystyrene latex particles containing 75% chloromethyl groups prepared by emulsion polymerization of styrene and chloromethyl styrene were washed and deionized on a mixed bed resin as follows: 2 g of AG501•x8 mixed bed resin (Biorad, Richmond, Cal., USA) was taken in a 30ml corex glass test tube. To the tube 2ml deionized water and 1ml of 10% suspension of the resin was added. The mixture was shaken for one hour at room temperature. The suspension was then filtered through a scintered glass funnel to collect the beads as filtrate. The resin bed was then washed with 1ml of deionized water. The wasing and deionization on the mixed bed resin was repeated once. The final washed material was stored at 4°C in a glass tube. This procedure is routeinely used to deinoize and wash latex particles.

The immobilization of primary amine containing oligonucelotides was done as follows: 400 microliters of 10 mM sodium borate (pH8.3) was taken in a microfuge tube (1.5ml) and 100 microliters of the washed latex particles (approximately 2mg) were added followed by 10 microliters of the oligonucleotides at 1 microgram per microliter in the same buffer. The mixture was shaken at room temperature for 4 hours. The unreacted chloromethyl groups were blocked by adding 500 microliters of 100mM glycine, 0.1% "TWEEN 20" at room temperature for 18 hours. The suspension was then centrifuged to remvoe unreacted materials and washed with 100mM glycine, "TWEEN 20" and 1% bovine serum albumin. The latex particles were then stored in the wash buffer at 4°C.

A similar method can be used to couple an aminolink (-NH$_2$ containing) oligonucleotide to any amine reactive solid support.


Example 6: Amplification of a Human Single Copy Gene Sequence and the Detection of the Product

This example was conducted out to demonstrate the feasibility of using an immobilized oligonucleotide primer for amplification. A typical 100 microliter reaction mixture contains the following:
1 microliter deoxynucleoside triphosphate mixture of all four nucleosides, 25mM each,
2 microliters of gelatin, 10 micrograms per microliter,
2 microliters of the soluble oligoprimers, 1 microgram per microliter,
10 microliter of the immobilized primer, x microliter of template (concentration can be as low as one picogram)
2.5 units of a thermostable DNA polymerase (1 microliter taq polymerase from New England Biolab) and ( 100-(x + 16) ) microliters of a buffer containing 50mM KCl, 10mM Tris pH 8.4, 2.5 mM MgCl$_2$.

The mixture was then processed through 30 to 35 repeats of the following heating-cooling cycles: the mixture was heated to 95°C then cooled to 40°C for annealing and then heated to 70°C for extension. At the end of the last cycle, the mixture was maintained at 70°C for 10 minutes to complete the last cycle. The samples were then analyzed on an agarose gel. The results are shown in Fig. 2.

The product of amplification from left to right using pss 737 as the sample (Geever et al, (1981), Proc. Nat. Acad. Sci., USA, 78, 5081-5085) is as follows:

| S*/pss | : both primers in solution |
|---|---|
| 6, 7* | : 7 labelled with rhodamine and in solution; 6 immobilized |
| 6*, 7 | : 6* labelled with rhodamine and in solution; 7 immobilied |
| S*/AA | : Normal beta-globin gene containing DNA test sample nucleic acid, both primers in solution and labelled |
| 6, 7*/AA | : 6 is immobilized; 7* labelled and in solution and normal beta-globin gene containing DNA |
| 6*, 7 AA | : 7 is immobilized: 6* is labelled and in solution and normal beta-globin gene containing DNA |
| S*/ss, 6, 7*/ss and 6*, 7/ss same as a the last three, except the test sample was from a patient with sickle cell anemia. | |

Ø X 174-Hae III is a marker nucleic acid. If one of the deoxy NTP's is labeled, e.g., with $^{32}$P direct counting of radioactivity before and after a restriction digestion will give the information about the sequence.

The sequence of the immobilized oligonucleotide 6 was the same as the WP primer of Example 1 with the addition of ten adenines at the 5' end.

The sequence of the other primer, 7, was the same as the CP primer of Example 1 with the addition of ten adenines at the 5' end.

The amount of the template used was about 1 picogram.


Example 7: The Specificity of amplification

The amplification is performed as described in Example 5 only with pss 737 using immobilized primers is shown in Fig. 3. The same notations applied to Fig. 2 are again used. D stands for digestion with a restriction emdonuclease DdeI. Digestion of the nonimmobilized 110-130 bp fragment, shown in the lanes designated 1,2/D; 6,7/D; 6,2/D and 6*,7*/D, results in two fragments of equal length because the DdeI restriction site is centrally located. Digestion of the immobilized 110-130 bp fragment, shown in the lane designated 6,7*/D, results in a single soluble fragment and a second bead attached fragment of slower mobility.

Example 8: Use of Fluorescently Labelled Oligoprimers for Amplification

The procedure was identical to that decribed in Example 6, except a rhodamine labelled oligoprimer was used instead of an unmodified soluble oligonucleotide primer. The labelled oligonucleotides were synthesized on a synthesiser (APPLIED BIOSYSTEMS, 380) and derivatized according to the protocol supplied by Applied Biosystems using the supplied chemcials. The sequences of the primer and the template used were the same as in Example 6.

Example 9: Amplification and Analyses of Chlamydia Trachomitis

Using the methods described in the previous examples that the modified oligonucleotides can be successfully used for the analysis of a specific sequence by sample amplification, one of the embodiments of the present invention was used, for the analysis of a sexually transmitted organism. The oligonucleotide primers were chosen such that they correspond to the conserved region of a common protein of chlamydia trachomitis to cover all serotypes and the amplified sequence should have enough variability to provide information about specific serotypes. The sequences which were chosen for thce present example correspond to the coding sequence nucleotide residues of a major outer membrane protein of the organism. The sequence corresponds to residues 1293 to 1313 for one primer and complementary to residues 1527 to 1547 for the other primer (R.S. Stephens, J. Bacteriology, 169, 3879 (1987)). This will produce a 255 bp fragment of DNA as the amplified product. The conditions used for amplification were identical to that used in Example 6 and a gel electrophoresis analysis showed the formation of the expected product.

Sequences of the 21 mers are as follows:

5′ amino- CAA GCA AGT TTA GCT CTC TCT and

5′ amino- AGA TTT TCT AGA TTT CAT CTT

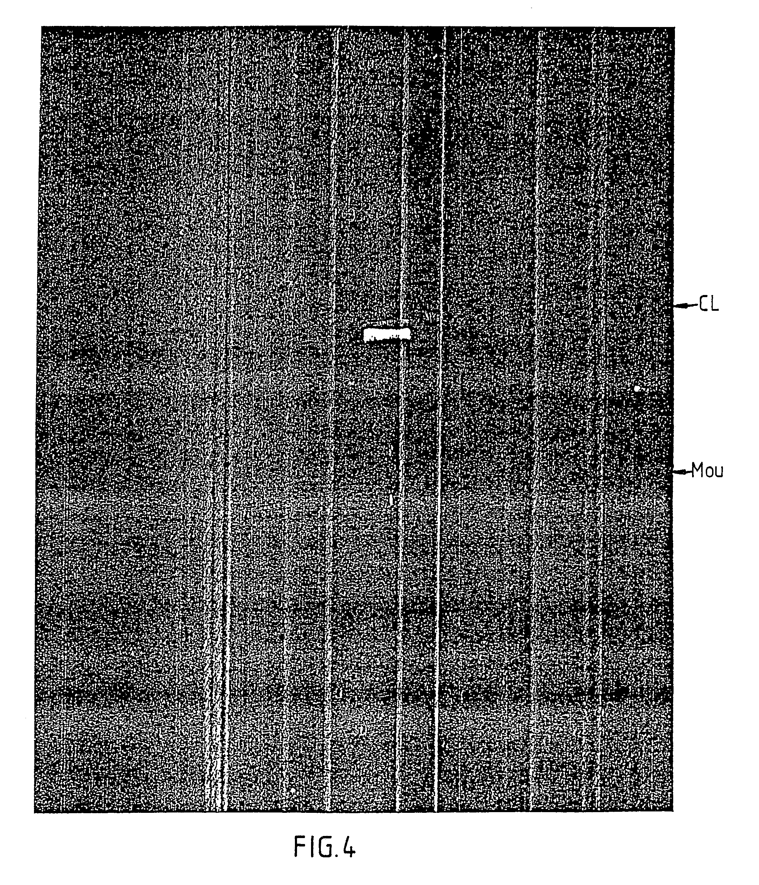

The primers were modified and labelled with rhodamine as previously described. The primers were added to a sample containing total genomic Chlamydia DNA. After amplification, unreacted labelled oligonucleotide was separated on an agarose gel by electrophoresis. Mouse DNA was used as a control since the Chlamydia was grown in host mouse cells. The colored band and the corresponding control were cut out and photographed as shown in Fig. 4.

Simple centrifugation or filtration may also be used for the separation of the unincorporated colored (labeled) oligionucleotide primers.

The immobilized labelled primers provide a sensitive, specific, easily analyzable assay that can be fully automated. A patient sample with the addition of immobilized or immobilizable labelled primers, subjected to thermocycling can provide an assay for rapid clinical diagnosis.

It will be appreciated that the instant specification and claims are set forth by way of illustration and not limitation, and that various modifications and changes may be made without departing from the spirit and scope of the present invention.

## Claims

1. A method for amplifying specific target nucleic acid sequences, preferably oligonucleotides, in a sample, the method characterized by

(a) contacting under hybridization conditions (i) a first primer nucleic acid and a second primer nucleic acid, one of said first or second primer nucleic acids being immobilized or immobilizable, preferably to a solid substance, the other is either immobilized or immobilizable or labeled, with (ii) a test sample containing target nucleic acid sequences,

(b) contacting the resultant product of step (a) with an extender enzyme and at least one nucleotide residue, preferably nuclectide triphosphate, for extension under conditions to elongate the resultant hybridized primer nucleic acids, preferably in a direction opposite to said solid substrate,

(c) denaturing the product of step (b), to produce immobilized target nucleic acid sequences, and

(d) repeating at least once a cycle of steps (a), (b) and (c) with the product of step (c) of the previous cycle being used in place of (a)(ii).

2. A method for detecting specific target nucleic acid sequences, preferably oligonucleotides, in a sample, the method characterized by

(a) contacting under hybridization conditions (i) a first primer nucleic acid a second primer nucleic acid, one of said first or second primer nucelic acids being immobilized or immobilizable, preferably to a solid substrate, the other is either immobilized or immobilizable or labeled, with (ii) a test sample suspected of containing target nucleic acid sequences,

(b) contacting the resultant product of step (a) with an extender enzyme and at least one nucleotide residue, preferably labelled nucleoside triphosphate, for extension under conditions to elongate the resultant hybridized nucleic acids, preferably in a direction opposite to said solid substrate,

(c) denaturing the product of step (b), to produce immobilized target nucleic acid sequences,

(d) repeating at least once a cycle of steps (a), (b) and (c) with the product of step (c) of the previous cycle being used in place of (a)(ii), and.

(e) determining if an amplified sequence is present.

3. A method according to claim 2, wherein step (e) is characterized by subjecting to hybridization conditions the product of step (d) of claim 2, with a labeled probe hybridizable with the sequence being tested for and determining if in fact hybridization has occurred.

4. A method according to claim 2, wherein detection is conducted by determining if there is a difference in a physical property for the specific target nucleic acid sequences, wherein said difference in a physical property is determined by electrophoresis, centrifugation, gel permeation chromatography or microscopy.

5. A method for detecting the presence or absence of at least one specific nucleic acid sequence in a sample containing a nucleic acid or combination of nucleic acids, or distinguishing between two different sequences in said sample, wherein the sample is suspected of containing said sequence or sequences, in a mixture, which process is characterized by

(a) treating the sample mixture with at least one immobilized oligonucleotide primer for at least one of each strand of each different specific sequence, under hybridizing conditions such that for each strand of each different sequence to which a primer is hybridized an extension product of each primer is synthesized which is complementary to each nucleic acid strand, wherein said primer or primers are selected so as to be sufficiently complementary to each strand of each specific sequence to hybridize therewith such that the extension product synthesized from one primer, when it is separated from its complement, can serve as a template for synthesis of the extension product of the other primer, and preferably at least one of said oligoprimers is a labelled oligonucleotide,

(b) treating the product of step (a) under denaturing conditions to separate the primer extension products from their templates,

(c) repeating step (a) with the product of step (b) resulting in amplification of the specific nucleic acid sequence or sequences which is an immobilized end product, wherein the immobilized end product is analyzed.

6. A kit for performing the method of claim 1 or 2, characterized by one or more containers containing

(a) an immobilized or immobilizable primer nucleic acid,.

(b) an immobilized, immobilizable or labeled nucleic acid,

(c) an extender enzyme,

(d) at least one nucleotide residue for extension and preferably

(e) a labeled hybridizable oligonucleotide specific for the detection of the amplified sequence.

7. A method according to claim 2, which is further characterized by analyzing the immobilized end product, by

(i) labeling the immobilized end product before or after separation of the end product from the sample mixture, and

(ii) analyzing the labeled end product.

8. A method for detecting the presence or absence of at least one specific nucleic acid sequence in a sample containing a nucleic acid or combination of nucleic acids, or distinguishing between two different sequences in said sample, wherein the sample is suspected of containing said sequence or sequences, in a mixture, which a process is characterized by

(a) treating the sample mixture with at least one immobilizable oligonucleotide primer, preferably a labeled oligonucleotide, for at least one of each strand of each different specific sequence, under hybridizing conditions such that for each strand of each different sequence to which a priner is hybridized an extension product of each primer is synthesized which is complementary to each nucleic acid strand, wherein said primer or primers are selected so as to be sufficiently complementary to each strand of each specific sequence to hybridize therewith such that the extension product synthesized from one primer, when it is separated from its complement, can serve as a template for synthesis of the extension product of the other primer,

(b) treating the product of step (a) under denaturing conditions to separate the primer extension products from their templates,

(c) repeating step (a) with the product of step (b) resulting in amplification of the specific nucleic acid sequence or sequences which is an immobilizable end product, and

(d) analyzing the immobilizable end product.

9. A method according to claim 8, which is further characterized by analyzing the immobilizable end product, said analyzing by

(i) separating the immobilized end product from the sample mixture,

(ii) immobilizing the end product,

(iii) adding to the product of step (ii) a label for binding to the immobilized end product and

(iv) analyzing the labeled end product.

10. A method according to claim 8, which is further characterized by analyzing the immobilizable end product, said analyzing by

(i) adding to the end product from step (c) a label for binding to the immobilized end product,

(ii) immobilizing the labeled end product,

(iii) separating the labeled immobilized end product from the sample mixture and

(iv) analyzing the labeled end product.

FIG.1

ØX174 Hae III

S* /PSS

6, 7* /PSS

6*, 7/PSS

S* /AA

6, 7*/AA

6*, 7/AA

S* /SS

6, 7*/SS

6*, 7/SS

FIG. 2

ØX174 Hae III
1,2
1,2/D
6,7
6,7/D
6,7
6,7/D
6,2
6,2/D
6,2
6,2/D

6,7*
6,7*/D
6,7*
6,7*/D
ØX174 Hae III

FIG.3

FIG.4